# EUROPEAN PATENT APPLICATION

(11) **EP 4 180 201 A1**
(43) Date of publication of application: **17.05.2023**
(21) Application number: 21207708.5
(22) Date of filing: 11.11.2021
(51) Int. Cl.: B29C 39/00, B29C 39/26, B29C 39/42, B29C 33/00, B29C 33/10, B29C 33/30, B29C 33/40, B29C 33/42, B29C 35/08, B29C 67/24

(54) **METHOD AND APPARATUS FOR REACTION INJECTION MOLDING OF STRUCTURED COMPONENTS USING A UV-CURABLE RESIN**

(71) Applicant: Robert Bosch Gesellschaft für medizinische Forschung mbH, 70376 Stuttgart (DE)
(72) Inventor: Zandi Shafagh, Reza, 18347 Täby, Stockholm (SE); Lauschke, Volker, 11544 Stockholm (SE)
(74) Representative: Bee, Joachim

(57) **Abstract**

There is disclosed a method (10) for reaction injection molding of a structured component (22), wherein the method (10) comprises the steps of: closing (S10) a mold (28) in order to provide a cavity (30) adapted to form the component (22), wherein the mold (28) comprises a negative shape of the component (22); injecting (S14) an reaction injection molding resin (84) at a low packing pressure into the cavity (30) in order to fill the cavity (30); curing (S16) the reaction injection molding resin (84) in the cavity (30) using UV-light (36) through a transparency in the mold (28); opening (S18) the mold (28); and demolding (S20) the cured component (22). Also, a corresponding component (22), apparatus (20; 70) and system (80) are disclosed.

## Description

The present invention relates to a method and an apparatus for reaction injection molding of structured components using a UV-curable resin.

### Background

Micro- and nanostructuring enables precise engineering of compact devices for a plethora of applications in electronics (see REF1, REF2 from the attached list of references), optics (see REF3), photonics (see REF4) and biomedicine (see REF5, REF6). While a diverse range of cleanroom-based nanopatterning methods are available, such methods are complex, costly and mainly suited for the miniaturization of integrated circuits (ICs). Photo- (normal/deep/extreme UV, x-ray) lithography methods have been consistently positioned at the forefront for very large-scale integration (VLSI) (see REF7). However, these methods are highly complex in the exposure system and limited in resolution footprint by light diffraction (see REF8) and photomask patterning (see REF9). Electron beam lithography is capable of ultra-high resolution pattern definition, yet its utilization is impeded by the very slow pace of processing due to the serial exposure (see REF10). Nanoimprint lithography (NIL) is another high-resolution nanofabrication method based on using a stamp for direct micro- and nanopatterning, however, the defectivity-prone replication has limited its widespread utility (see REF11). Importantly, all the above-noted nanofabrication methods are mainly tuned for silicon device manufacturing and commonly require additional multi-step processing, such as metal deposition and etching processes. In addition, lithography-based approaches deal with photo or e-beam resists with limited thickness, from tens of nanometer to hundreds of microns that in turn results in limited lateral resolution and aspect ratio (see REF12, REF13). Thus, the thermoplastic and thermosetting polymer resins used in these processes are mainly utilized as sacrificial layers in bulk silicon wafers (see REF14) and, therefore, the formation of cm-sized polymer devices are not generally devised in such settings. Moreover, due to the high-cost of fabrication, such cleanroom-originating devices are hardly amenable for disposable use (see REF15).

Soft lithography was introduced as an alternative nanofabrication method based on the casting of polydimethylsiloxane (PDMS) on a master mold (see REF16, REF17) and has persisted as the dominant fabrication method in the microfluidic domain for more than two decades due to its low capital cost, ease of use and biocompatibility of PDMS (see REF18). However, casting is merely designed for prototyping of microfluidic devices and is essentially not scalable due to long cycle times and incompatibility with batch fabrication (see REF19). These limitations have resulted in an increasing gap between academic research and commercial product dissemination (see REF20, REF21). In addition, the intrinsic shortcomings of PDMS including absorption of hydrophobic molecules (see REF22), leaching of monomers (see REF23, REF24) and evaporation of medium (see REF25) raise concerns about the suitability of this material for pharmacological and toxicological applications.

Injection Molding (IM) (see REF26, REF27) machinery has been adapted and implemented for micro- and nanostructuring of polymer devices on industrial scales. Micro-injection molding (µIM) is currently the only scalable platform for microstructuring of polymers at the whole-device level (see REF28). Nevertheless, the intrinsic properties of thermoplastic materials, such as the high degree of viscosity (see REF29), have imposed demanding and harsh processing conditions in terms of injection and packing pressure (>100 MPa) (see REF30), as well as processing temperature (>200°C) (see REF31). Furthermore, the premature solidification of polymer melts inside micro- and nanocavities necessitates variotherm process (see REF32) adding further to the complexity of the system, and the extensive shrinkage of thermoplastics drastically impairs replication fidelity. Lastly, different injection shot volumes (see REF33) designed for either macro or micro molding complicates the combination of divergent feature sizes in a single injection process. Such limitations and complexities have restricted the use of µIM and significantly raised the capital investment for polymer nanofabrication on cm-sized device levels (see REF34). Other polymer microstructuring methods such as hot-embossing and roll-to-roll imprinting suffer from long cycle-time, noticeable residual thermal stress, and cumbersome demolding (see REF35, REF36) or are limited mainly to surfaces (see REF37).

Reaction Injection Molding (RIM) of micro polymer parts has been introduced in the 1980s (see REF38). Nevertheless, due to the high shrinkage rates (> 20%), long cycle time and difficulty in monomer resin mixing attributed to the thermosetting materials, the platform was not successfully commercialized and, therefore, µIM has remained the dominant platform for the fabrication polymer microdevices.

### Summary of the disclosure

According to an aspect of the disclosure, there is provided a method for reaction injection molding of a structured component, wherein the method comprises the steps of:
- closing a mold in order to provide a cavity adapted to form the component, wherein the mold comprises a negative shape of the component;
- injecting a UV-curable reaction injection molding resin at a low packing pressure into the cavity in order to fill the cavity;
- curing the reaction injection molding resin in the cavity using UV-light through a transparency in the mold;
- opening the mold; and
- demolding the cured component.

The advantages that can be achieved with this new methodology are explained further down. In an exemplary embodiment, air is allowed to escape from the mold during the step of injecting.

In an exemplary refinement, between the step of closing and the step of injecting the following step is performed: evacuating air from the cavity in order to provide a partial vacuum in the cavity. This allows for a very low active packing pressure when injecting the reaction injection molding resin down to, e.g., a low active packing pressure of 0 mbar which means that atmospheric pressure is sufficient to drive the resin into the mold.

In a further exemplary refinement, the temperature of the reaction injection molding resin is not actively changed substantially during the molding process and/or the temperature is maintained in a range between 0°C and 50°C. This refinement allows for relaxed and simplified process control. It is noted that a constant temperature and/or any range temperature is not necessary. However, this can be beneficial if an additional bonding process is required.

In a further exemplary refinement, the reaction injection molding resin is a UV-curable prepolymer with a low viscosity at room temperature. This refinement allows for relaxed and simplified process control. The low viscosity allows for a good filling of the cavity at a low packing pressure during the injection. In an exemplary embodiment, the thermoset polymerization features a delayed gelation and low viscosity in order to improve the forming of fine structures.

In a further exemplary refinement, the reaction injection molding resin is thiol-ene-based or thiol-ene-epoxy-based. This type of resin was identified as beneficial during trials that have been performed using this new methodology.

According to a further aspect, there is provided a component obtained by a method as described above.

According to a further aspect, there is provided an apparatus for reaction injection molding of a structured component, wherein the apparatus comprises a first mold part and a second mold part which are adapted, in a closed configuration, to form a mold that contains a cavity adapted to form the component, the mold comprising an injection port adapted to receive a UV-curable reaction injection molding resin at a low packing pressure to fill the cavity, wherein at least one of the first and second mold parts is adapted to provide a transparency adapted to allow for curing the reaction injection molding resin inside the mold using UV-light, wherein the first mold part and a second mold, in the closed configuration, are held together by a clamping device.

The advantages that can be achieved with this new methodology are explained further down. In an exemplary embodiment, it is sufficient for the clamping device to be able to provide a mild clamping force due to the low packing pressure used during the injection. In a further exemplary embodiment, the injection port is used as an evacuation port in order to evacuate air from the cavity before the reaction injection mold resin is injected.

In an exemplary refinement, the mold comprises an evacuation port adapted for evacuation of air from the cavity. This allows for a very low active packing pressure when injecting the reaction injection mold resin even down to 0 mbar which means that atmospheric pressure is sufficient to drive the resin into the mold.

In a further exemplary refinement, the first mold part is formed from any machinable UV-transparent material, such as polymethylmethacrylate or comprises polymethylmethacrylate to provide the transparency.

According to a further aspect, there is provided a system for reaction injection molding of a structured component, wherein the system comprises an apparatus as described above, a UV-light source, a reservoir for the UV-curable reaction injection molding resin in fluid connection to the injection port, and an optional vacuum pump in fluid connection to the evacuation port.

As one aspect of the presented disclosure the development a new paradigm for nanostructuring of polymer devices based on thiol-ene-(epoxy) click chemistry is presented. Some of the benefits that can be achieved with the presented teachings are allowing for a rapid polymerization via UV exposure and a shrinkage of less than 1%, which renders these resins highly suitable for a reaction injection molding process. The present teachings enable the fabrication of devices with macrocomponents and nanotopograhies with structures down to 50nm, all implemented in a single fabrication step.

Furthermore, the present teachings offer the capability of nanostructuring both flexible and stiff materials, and its mechanical properties can be manipulated to fabricate high aspect ratio (HAR) and complex 3D structures via controlled collapse. The present teachings are useful for the structuring of polymer devices with diverse applications in optics and biomedicine. Specifically, the formation of vivid structural colors on polymer device level and the production of commercial-grade biocompatible microfluidic devices that allow for the culture of organotypic patient-derived primary human liver models with superior molecular phenotypes are considered.

As a further aspect of the presented disclosure teachings for the injection molding of thermosetting resins for the structuring of both stiff (GPa) and flexible (MPa) polymer devices comprised of complex multiscale features is presented. The present teachings enable further a single-step fabrication of cm-sized thermoset chips containing mm-sized world-to-chip tube connectors and chambers, µm-sized meandering channels and submicron and nanosized topographies, including high-aspect-ratio (HAR) pillars, wells and gratings, with feature sizes down to 50 nm. This may be achieved without the implementation of variotherm processes and despite using zero packing pressure with short cycle times, e.g. of 10-100s or 20-120s.

The present teachings further enable applications 1) in optics for the scalable fabrication of vivid and stable structural colors, on both part and device levels, and 2) in biomedicine via the multi-planar structuring of commercial-grade organ-on-a-chip devices in which 3D primary human tissue models exhibit high cell viability and superior molecular phenotypes. The present teachings further enable an ultra-high-resolution structuring and the modularity of mechanical properties in tandem with low tooling cost and suitability across a wide range of feature sizes. The present teachings further enable a facile and scalable polymer nanostructuring paradigm, enabling the successful translation of nanostructured thermoset devices from research to practical use.

In an exemplary embodiment, the first mold part and/or the second mold part comprises two or more separable elements. In a further exemplary embodiment, one or more further mold parts are present in addition to the first mold part and the second mold part in order to form the mold. In a further exemplary embodiment, at least one of the mold parts includes one or more respective mold inserts. In a further exemplary embodiment, the structured component comprises one or more of macro-, micro- or nanostructures. In a further exemplary embodiment, a multiplanar and/or multiscalar structured component is produced in a single injection shot that fills the cavity at least substantially completely.

In an exemplary embodiment, the closing of the mold comprises using a dedicated fixture or elements of a dedicated fixture. In a further exemplary embodiment, the closing of the mold comprises using a separate cover mold. In a further exemplary embodiment, if the mold is supported by an element of a fixture, the transparency is also provided in a part of the fixture.

In an exemplary embodiment the low viscosity is in a range selected from the group of 1 mPas to 10,000 mPas, 10 mPas to 8,000 mPas, 100 mPas to 7,000 mPas, 250 mPas to 6,000 mPas, or 500 mPas to 5,000 mPas.

In an exemplary embodiment the partial vacuum is in a range selected from the group 0.1 mbar to 100 mbar, 0.25 mbar to 75 mbar, 0.5 mbar to 50 mbar, 0.75 mbar to 25 mbar, or 1 mbar to 10 mbar, with atmospheric pressure as a reference point. Thus, e.g., a partial vacuum of 10 mbar is 10 mbar below atmospheric pressure.

In an exemplary embodiment the low packing pressure is smaller than one of 100 bar, 50 bar, 25 bar or 10 bar, with atmospheric pressure as a reference point. Thus, e.g., a low packing pressure of 0 bar is atmospheric pressure.

In an exemplary embodiment the low temperature is in a range selected from the group of 0 °C to 100 °C, 3 °C to 90 °C, 6 °C to 80 °C, 9 °C to 70 °C, 12 °C to 80 °C, or 15 °C to 50 °C.

In an exemplary embodiment the room temperature is in a range selected from the group of 10 °C to 40 °C, 15 °C to 35 °C, or 20 °C to 30 °C.

In an exemplary embodiment the mild clamping force is smaller than one of 0.1 ton-force, 0.075 ton-force, 0.05 ton-force, 0.025 ton-force, or 0.01 ton-force.

In an exemplary embodiment at least one of the first mold part or the second mold part comprises master structures that are negative to structures of the component, wherein the master structures are embodied in at least one of machinable organic and/or of inorganic materials, including silicon, fused silica, polydimethylsiloxane, cyclic olefin copolymer, poly (methyl methacrylate), polycarbonate, polystyrene, polypropylene, polytetrafluoroethylene, aluminum, quartz, soda lime, glass metals or steel.

In an exemplary embodiment the resin is mixed and degassed prior to injection to minimize the formation of bubbles/defects.

### Brief description of the drawings

Examples of embodiments of the invention are shown in the drawing and explained in more detail in the following description. In the figures,
Figure 1 shows a flow diagram of a method for reaction injection molding of a structured component according to an embodiment;
Figure 2 schematically shows an apparatus for reaction injection molding of a structured component according to a first embodiment in a closed configuration;
Figure 3 schematically shows the apparatus according to the first embodiment in an exploded or open configuration and showing a molded component;
Figure 4 schematically shows an apparatus for reaction injection molding of a structured component according to a second embodiment; and
Figure 5 schematically shows a system using the apparatus according to the second embodiment.

### Detailed description

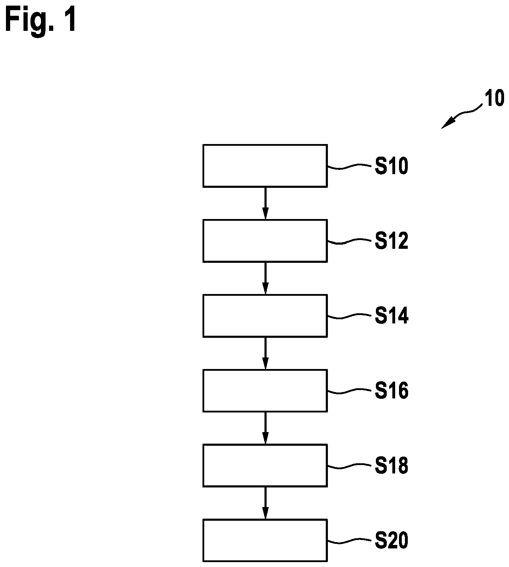

Figure 1 shows a method 10 for reaction injection molding of a structured component using a UV-curable resin. The method 10 starts with closing S10 a mold in order to provide a cavity adapted to form the component, wherein the mold comprises a negative shape of the component.

In an optional step S12 air is evacuated from the cavity in order to provide a partial vacuum in the cavity. It is pointed out that it is possible to fill the cavity purely by the packing pressure of the reaction injection molding resin.

The method 10 continues with injecting S14 a UV-curable reaction injection molding resin at a low packing pressure into the cavity in order to fill the cavity. Optionally, this step comprises allowing air in the mold to escape through an opening in the mold.

After the mold has been filled, the reaction injection molding resin is cured S16 in the cavity using UV-light through a transparency in the mold. After the curing step is complete, the mold is opened S18 and the cured structured component is demolded S20.

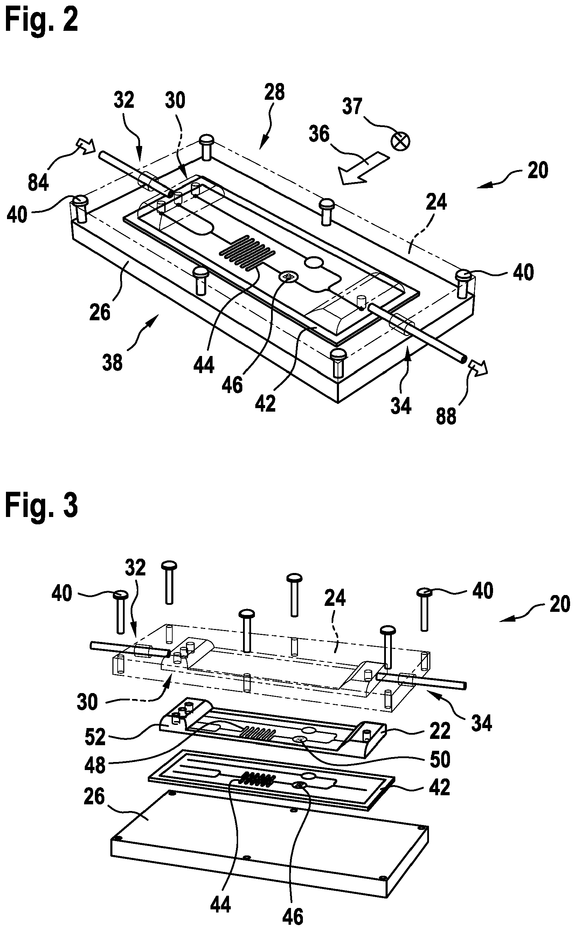

Figure 2 schematically shows an apparatus 20 for reaction injection molding of a structured component 22 (see Figure 3) using a UV-curable resin according to a first embodiment in a closed configuration.

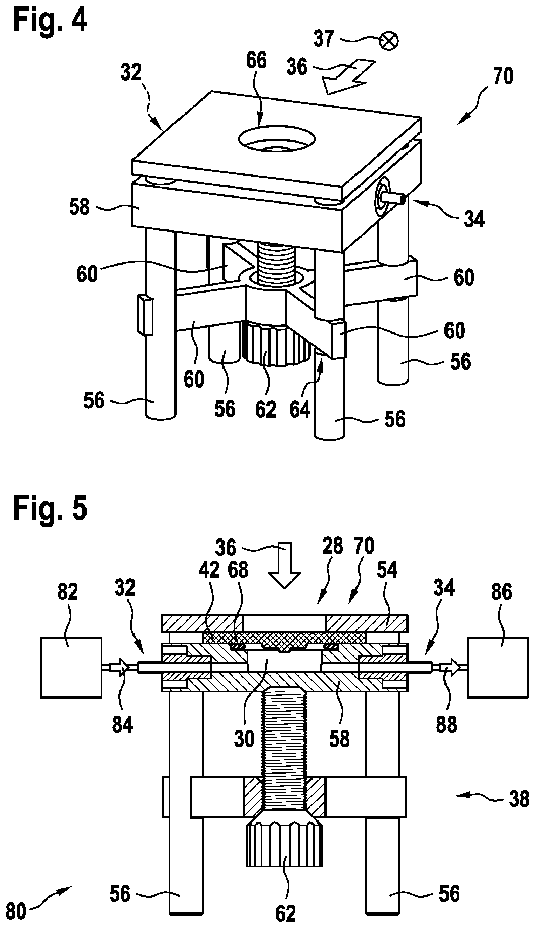

The apparatus 20 comprises a first mold part 24 and a second mold part 26 which are adapted, in the shown closed configuration, to form a mold 28 that contains a cavity 30 adapted to form the component 22. The mold 28 comprises an injection port 32 adapted to receive a UV-curable reaction injection molding resin 84 (see Figure 5) at a low packing pressure to fill the cavity 30 and an evacuation port 34 adapted for evacuation of air 88 (see Figure 5) from the cavity 30.

At least one of the first and second mold parts 24, 26, here the first mold part 24, is adapted to provide a transparency adapted to allow for curing the reaction injection molding resin 84 inside the mold 28 using UV-light 36 (symbolized by the arrow) from a UV-light source 37. The first mold part 24 and the second mold part 26, in the closed configuration, are held together by a clamping device 38. In this embodiment, the clamping device 38 is provided by six screws 40 that go through the first mold part 24 into screw threads (not shown) in the second mold part 26. Only two screws 40 show their reference numeral in order to not obscure the figure.

The second mold part 26 comprises a mold insert 42 on which master-microstructures 44 and master-nanostructures 46 are provided that form corresponding microstructures 48 and nanostructures 50 on the structured component 22. It is noted that the structured component 22 has also macrostructures 52 that are formed in corresponding master-macrostructures in the first mold part 24.

In this embodiment the master-nanostructures 46 are configured in order to provide nanostructures 50 as diffraction gratings in order to implement color generation based on Bragg-scattering on the component 22. In an exemplary embodiment, polymer gratings are structured with varying width and interspacing from 400 nm to 700 nm with 100 nm increments, leading to constructive interference of incident light.

Figure 3 schematically shows the apparatus 20 according to the first embodiment in an exploded or open configuration and showing the structured molded component 22.

Figure 4 schematically shows an apparatus 70 for reaction injection molding of a structured component 22 according to a second embodiment. Those elements known from the first embodiment that have a corresponding function in the second embodiment are not be explained again and are given the previously introduced reference numerals.

The apparatus 70 has a fixture 54 comprising a first fixture part 54 rigidly connected to four columns 56 and a second fixture part 58 slidably arranged on the columns 56. The first fixture part 54 has an opening 66 that allows UV-light 36 to reach the transparency of the first mold part 24 or the combined second mold part 26 and mold insert 42.

The apparatus 70 further has a clamping device 38 which has four arms 60 in a cross-like shape and a clamping screw 62 arranged in the middle of the four arms 60. The clamping device 38 sits in recesses 64 that are provided in the columns 56. An O-ring 68 provides a seal

Figure 5 schematically shows a system 80 for reaction injection molding of a structured component 22 using a UV-curable resin 84 and using the apparatus 70 according to the second embodiment.

The system 80 comprises the apparatus 70, a source 37 for UV-light 36, a reservoir 82 for the UV-curable reaction injection molding resin 84 in fluid connection to the injection port 32, and an optional vacuum pump 86 in fluid connection to the evacuation port 34.

The above detailed description includes references to the accompanying drawings, which form a part of the detailed description. The drawings show, by way of illustration, specific embodiments in which the invention may be practiced. These embodiments are also referred to herein as "examples." Such examples can include elements in addition to those shown or described. However, the present inventors also contemplate examples in which only those elements shown or described are provided. Moreover, the present inventors also contemplate examples using any combination or permutation of those elements shown or described (or one or more aspects thereof), either with respect to a particular example (or one or more aspects thereof), or with respect to other examples (or one or more aspects thereof) shown or described herein.

In this document, the terms "a" or "an" are used, as is common in patent documents, to include one or more than one, independent of any other instances or usages of "at least one" or "one or more." In this document, the term "or" is used to refer to a nonexclusive or, such that "A or B" includes "A but not B," "B but not A," and "A and B," unless otherwise indicated. In this document, the terms "including" and "in which" are used as the plain-English equivalents of the respective terms "comprising" and "wherein." Also, the terms "including" and "comprising" are open-ended, that is, a system, device, article, composition, formulation, or process that includes elements in addition to those listed after such a term are still deemed to fall within the scope of subject matter discussed. Moreover, such as may appear in a claim, the terms "first," "second," and "third," etc. are used merely as labels, and are not intended to impose numerical requirements on their objects.

The above description is intended to be illustrative, and not restrictive. For example, the above-described examples (or one or more aspects thereof) may be used in combination with each other. Other embodiments may be used, such as by one of ordinary skill in the art upon reviewing the above description. The abstract is provided to allow the reader to quickly ascertain the nature of the technical disclosure. It is submitted with the understanding that it will not be used to interpret or limit the scope or meaning of a claim. Also, in the above description, various features may be grouped together to streamline the disclosure. This should not be interpreted as intending that an unclaimed disclosed feature is essential to any claim. Rather, inventive subject matter may lie in less than all features of a particular disclosed embodiment. The following aspects are hereby incorporated into the description as examples or embodiments, with each aspect standing on its own as a separate embodiment, and it is contemplated that such embodiments may be combined with each other in various combinations or permutations.

### List of references

(REF1) Sangwan, V. K.; Hersam, M. C. Neuromorphic Nanoelectronic Materials. Nat Nanotechnol 2020, 15 (7), 517-528. https://doi.org/10.1038/s41565-020-0647-z.
(REF2) Ren, Y.; Zou, Y.; Liu, Y.; Zhou, X.; Ma, J.; Zhao, D.; Wei, G.; Ai, Y.; Xi, S.; Deng, Y. Synthesis of Orthogonally Assembled 3D Cross-Stacked Metal Oxide Semiconducting Nanowires. Nat Mater 2020, 19 (2), 203-211. https://doi.org/10.1038/s41563-019-0542-x.
(REF3) Lee, W.; Zhou, Z.; Chen, X.; Qin, N.; Jiang, J.; Liu, K.; Liu, M.; Tao, T. H.; Li, W. A Rewritable Optical Storage Medium of Silk Proteins Using Near-Field Nano-Optics. Nat Nanotechnol 2020, 15 (11), 941-947. https://doi.org/10.1038/s41565-020-0755-9.
(REF4) Arrazola, J. M.; Bergholm, V.; Brádler, K.; Bromley, T. R.; Collins, M. J.; Dhand, I.; Fumagalli, A.; Gerrits, T.; Goussev, A.; Helt, L. G.; Hundal, J.; Isacsson, T.; Israel, R. B.; Izaac, J.; Jahangiri, S.; Janik, R.; Killoran, N.; Kumar, S. P.; Lavoie, J.; Lita, A. E.; Mahler, D. H.; Menotti, M.; Morrison, B.; Nam, S. W.; Neuhaus, L.; Qi, H. Y.; Quesada, N.; Repingon, A.; Sabapathy, K. K.; Schuld, M.; Su, D.; Swinarton, J.; Szava, A.; Tan, K.; Tan, P.; Vaidya, V. D.; Vernon, Z.; Zabaneh, Z.; Zhang, Y. Quantum Circuits with Many Photons on a Programmable Nanophotonic Chip. Nature 2021, 591 (7848), 54-60. https://doi.org/10.1038/s41586-021-03202-1.
(REF5) Tinazli, A.; Piehler, J.; Beuttler, M.; Guckenberger, R.; Tampé, R. Native Protein Nanolithography That Can Write, Read and Erase. Nat Nanotechnol 2007, 2 (4), 220-225. https://doi.org/10.1038/nnano.2007.63.
(REF6) Divine, R.; Dang, H. V.; Ueda, G.; Fallas, J. A.; Vulovic, I.; Sheffler, W.; Saini, S.; Zhao, Y. T.; Raj, I. X.; Morawski, P. A.; Jennewein, M. F.; Homad, L. J.; Wan, Y.-H.; Tooley, M. R.; Seeger, F.; Etemadi, A.; Fahning, M. L.; Lazarovits, J.; Roederer, A.; Walls, A. C.; Stewart, L.; Mazloomi, M.; King, N. P.; Campbell, D. J.; McGuire, A. T.; Stamatatos, L.; Ruohola-Baker, H.; Mathieu, J.; Veesler, D.; Baker, D. Designed Proteins Assemble Antibodies into Modular Nanocages. Science 2021, 372 (6537), eabd9994. https://doi.org/10.1126/science.abd9994.
(REF7) Moreau, W. M. Semiconductor Lithography, Principles, Practices, and Materials. 1988. https://doi.org/10.1007/978-1-4613-0885-0.
(REF8) Rodgers, P. What Diffraction Limit? Nat Nanotechnol 2009, 4 (5), 280-280. https://doi.org/10.1038/nnano.2009.94.
(REF9) Love, J. C.; Wolfe, D. B.; Jacobs, H. O.; Whitesides, G. M. Microscope Projection Photolithography for Rapid Prototyping of Masters with Micron-Scale Features for Use in Soft Lithography. Langmuir 2001, 17 (19), 6005-6012. https://doi.org/10.1021/la010655t.
(REF10) Ito, T.; Okazaki, S. Pushing the Limits of Lithography. Nature 2000, 406 (6799), 1027-1031. https://doi.org/10.1038/35023233.
(REF11) Guo, L. J. Nanoimprint Lithography: Methods and Material Requirements. Advanced Materials 2007, 19 (4), 495-513. https://doi.org/10.1002/adma.200600882.
(REF12) Itani, T.; Kozawa, T. Resist Materials and Processes for Extreme Ultraviolet Lithography. Jpn J Appl Phys 2013, 52 (1R), 010002. https://doi.org/10.7567/jjap.52.01 0002.
(REF13) Gangnaik, A. S.; Georgiev, Y. M.; Holmes, J. D. New Generation Electron Beam Resists: A Review. ACS Publications 2017. https://doi.org/10.1021/acs.chemmater.6b03483.
(REF14) Li, W.; Huang, G.; Wang, J.; Yu, Y.; Wu, X.; Cui, X.; Mei, Y. Superelastic Metal Microsprings as Fluidic Sensors and Actuators. Lab Chip 2012, 12 (13), 2322-2328. https://doi.org/10.1039/c2lc40151g.
(REF15) Faustino, V.; Catarino, S. O.; Lima, R.; Minas, G. Biomedical Microfluidic Devices by Using Low-Cost Fabrication Techniques: A Review. J Biomech 2016, 49 (11), 2280-2292. https://doi.org/10.1016/j.jbiomech.2015.11.031.
(REF16) Zhao, X.-M.; Xia, Y.; Whitesides, G. M. Soft Lithographic Methods for NanoFabrication. J Mater Chem 1997, 7 (7), 1069-1074. https://doi.org/10.1039/a700145b.
(REF17) Xia, Y.; Whitesides, G. M. Soft Lithography. Angewandte Chemie Int Ed 1998, 37 (5), 550-575. https://doi.org/10.1002/(sici)1521-3773(19980316)37:5<550::aid-anie550>3.0.co;2-g.
(REF18) Tanyeri, M.; Tay, S. Viable Cell Culture in PDMS-Based Microfluidic Devices. Methods Cell Biol 2018, 148, 3-33. https://doi.org/10.1016/bs.mcb.2018.09.007.
(REF19) Becker, H.; Gärtner, C. Polymer Microfabrication Technologies for Microfluidic Systems. Analytical and Bioanalytical Chemistry 2007, 390 (1), 89-111. https://doi. org/10.1007/s00216-007-1692-2.
(REF20) Berthier, E.; Young, E. W. K.; Beebe, D. Engineers Are from PDMS-Land, Biologists Are from Polystyrenia. Lab Chip 2012, 12 (7), 1224-1237. https://doi.org/10.1039/c2lc20982a.
(REF21) Becker, H. Mind the Gap! Lab on a Chip 2010, 10 (3), 271-273. https://doi.org/10.1039/b925993g.
(REF22) Toepke, M. W.; Beebe, D. J. PDMS Absorption of Small Molecules and Consequences in Microfluidic Applications. Lab Chip 2006, 6 (12), 1484-1486. https://doi.org/10.1039/b612140c.
(REF23) Carter, S.-S. D.; Atif, A.-R.; Kadekar, S.; Lanekoff, I.; Engqvist, H.; Varghese, O. P.; Tenje, M.; Mestres, G. PDMS Leaching and Its Implications for On-Chip Studies Focusing on Bone Regeneration Applications. Organs-on-a-chip 2020, 2, 100004. https://doi.org/10.1016/j.ooc.2020.100004.
(REF24) Regehr, K. J.; Domenech, M.; Koepsel, J. T.; Carver, K. C.; Ellison-Zelski, S. J.; Murphy, W. L.; Schuler, L. A.; Alarid, E. T.; Beebe, D. J. Biological Implications of Polydimethylsiloxane-Based Microfluidic Cell Culture. Lab on a Chip 2009, 9 (15), 2132-17. https://doi.org/10.1039/b903043c.
(REF25) Mukhopadhyay, R. When PDMS Isn't the Best. Anal Chem 2007, 79 (9), 3248-3253. https://doi.org/10.1021/ac071903e.
(REF26) Piotter, V.; Mueller, K.; Plewa, K.; Ruprecht, R.; Hausselt, J. Performance and Simulation of Thermoplastic Micro Injection Molding. Microsyst Technol 2002, 8 (6), 387-390. https://doi.org/10.1007/s00542-002-0178-6.
(REF27) Becker, H.; Heim, U. Hot Embossing as a Method for the Fabrication of Polymer High Aspect Ratio Structures. Sensors Actuators Phys 2000, 83 (1-3), 130-135. https://doi.org/10.1016/s0924-4247(00)00296-x.
(REF28) Attia, U. M.; Marson, S.; Alcock, J. R. Micro-Injection Moulding of Polymer Microfluidic Devices. Microfluidics and Nanofluidics 2009, 7 (1), 1-28. https://doi.org/10.1007/s10404-009-0421-x.
(REF29) Wang, L.; Li, Q.; Zhu, W.; Shen, C. Scale Effect on Filling Stage in Micro-Injection Molding for Thin Slit Cavities. Microsyst Technol 2012, 18 (12), 2085-2091. https://doi.org/10.1007/s00542-012-1545-6.
(REF30) Yang, C.; Su, L.; Huang, C.; Huang, H.-X.; Castro, J. M.; Yi, A. Y. Effect of Packing Pressure on Refractive Index Variation in Injection Molding of Precision Plastic Optical Lens. Advances in Polymer Technology 2011, 30 (1), 51-61. https://doi.org/10.1002/adv.20211.
(REF31) Santis, F. D.; Pantani, R. Development of a Rapid Surface Temperature Variation System and Application to Micro-Injection Molding. Journal of Materials Processing Tech. 2016, 237, 1-11. https://doi.org/10.1016/j.jmatprotec.2016.05.023.
(REF32) Su, Q.; Zhang, N.; Gilchrist, M. D. The Use of Variotherm Systems for Microinjection Molding. Journal of Applied Polymer Science 2015, 133 (9), n/a-n/a. https://doi.org/10.1002/app.42962.
(REF33) Yang, C.; Yin, X.-H.; Cheng, G.-M. Microinjection Molding of Microsystem Components: New Aspects in Improving Performance. Journal of Micromechanics and Microengineering 2013, 23 (9), 093001-093022. https://doi.org/10.1088/0960-1317/23/9/093001.
(REF34) Juster, H.; Aar, B.; Brouwer, H. A Review on Microfabrication of Thermoplastic Polymer-based Microneedle Arrays. Polym Eng Sci 2019, 59 (5), 877-890. https://doi.org/10.1002/pen.25078.
(REF35) Becker, H.; Gärtner, C. Polymer Microfabrication Technologies for Microfluidic Systems. Analytical and Bioanalytical Chemistry 2008, 390 (1), 89-111. (REF36) Shan, X.; Liu, Y. C.; Lam, Y. C. Studies of Polymer Deformation and Recovery in Micro Hot Embossing. Microsyst Technol 2008, 14 (7), 1055-1060. https://doi.org/10.1007/s00542-007-0486-y.
(REF37) Ahn, S. H.; Guo, L. J. High-Speed Roll-to-Roll Nanoimprint Lithography on Flexible Plastic Substrates. Advanced Materials 2008, 20 (11), 2044-2049. https://doi.org/10.1002/adma.200702650.
(REF38) Hagmann, P.; Ehrfeld, W.; Vollmer, H. Fabrication of Microstructures with Extreme Structural Heights by Reaction Injection Molding. Makromolekulare Chemie. Macromolecular Symposia 1989, 24 (1), 241-251. https://doi.org/10.1002/masy.19890240125.

## Claims

1. Method (10) for reaction injection molding of a structured component (22), wherein the method (10) comprises the steps of:
- closing (S10) a mold (28) in order to provide a cavity (30) adapted to form the component (22), wherein the mold (28) comprises a negative shape of the component (22);
- injecting (S14) an reaction injection molding resin (84) at a low packing pressure into the cavity (30) in order to fill the cavity (30);
- curing (S16) the reaction injection molding resin (84) in the cavity (30) using UV-light (36) through a transparency in the mold (28);
- opening (S18) the mold (28); and
- demolding (S20) the cured component (22).

2. Method (10) of claim 1, wherein between the step of closing (S10) and the step of injecting (S14) the following step is performed:
- evacuating (S12) air from the cavity (30) in order to provide a partial vacuum in the cavity (30).

3. Method (10) of any preceding claim, wherein the temperature of the reaction injection molding resin (84) is not actively changed substantially during the molding process and/or wherein the temperature is maintained in a range between 0°C and 50°C.

4. Method (10) of any preceding claim, wherein the reaction injection molding resin (84) is a UV-curable prepolymer with a low viscosity at room temperature.

5. Method (10) of any preceding claim, wherein the reaction injection molding resin (84) is thiol-ene-based or thiol-ene-epoxy-based.

6. Component (22) obtained by a method (10) of any preceding claim.

7. Apparatus (20; 70) for reaction injection molding of a structured component (22), wherein the apparatus (20; 70) comprises a first mold part (24) and a second mold part (26) which are adapted, in a closed configuration, to form a mold (28) that contains a cavity (30) adapted to form the component (22), the mold (28) comprising an injection port (32) adapted to receive a UV-curable reaction injection molding resin (84) at a low packing pressure to fill the cavity (30), wherein at least one of the first and second mold parts (24, 26) is adapted to provide a transparency adapted to allow for curing the reaction injection molding resin (84) inside the mold (28) using UV-light (36), wherein the first mold part (24) and the second mold part (26), in the closed configuration, are held together by a clamping device (38).

8. Apparatus (20; 70) of claim 7 wherein the mold (28) comprises an evacuation port (34) adapted for evacuation of air (88) from the cavity (30).

9. Apparatus (20; 70) of claim 7 or 8 wherein the first mold part (24) is formed from a machinable UV-transparent material, such as polymethylmethacrylate or comprises polymethylmethacrylate to provide the transparency.

10. System (80) for reaction injection molding of a structured component, wherein the system (80) comprises an apparatus (20; 70) according to any of claims 7 to 9, a UV-light source (37), a reservoir (82) for the UV-curable reaction injection molding resin (84) in fluid connection to the injection port (32), and an optional vacuum pump (86) in fluid connection to the evacuation port (34).
